# EUROPEAN PATENT SPECIFICATION

(11) **EP 3 603 548 B1**
(45) Date of publication and mention of the grant of the patent: **18.05.2022**
(21) Application number: 18771309.4
(22) Date of filing: 23.03.2018
(51) Int. Cl.: A61B 17/34, A61M 25/06, A61M 29/00, A61B 90/00

(54) **DILATOR**
DILATATOR
DILATATEUR

(30) Priority: 24.03.2017 WO PCT/JP2017/012024
(43) Date of publication of application: 05.02.2020
(73) Proprietor: Asahi Intecc Co., Ltd., Seto-shi, Aichi 489-0071 (JP)
(72) Inventor: FUSEYA, Yukihiro, Seto-shi, Aichi 489-0071 (JP); MAKI, Hideaki, Seto-shi, Aichi 489-0071 (JP); TAKAHASHI, Daiki, Seto-shi, Aichi 489-0071 (JP); SAWAI, Akira, Seto-shi, Aichi 489-0071 (JP); KITAI, Marina, Seto-shi, Aichi 489-0071 (JP)
(74) Representative: Winter, Brandl - Partnerschaft mbB
(86) International application number: PCT/JP2018/011673
(87) International publication number: WO 2018/174242

(56) References cited:
- WO-A1-91/07202
- DE-A1- 19 636 318
- JP-A- 2002 177 289
- JP-A- 2007 098 120
- JP-A- 2016 013 285
- US-A- 5 630 813
- US-A1- 2002 077 655
- US-A1- 2011 098 531
- US-A1- 2014 046 357
- US-A1- 2014 046 357

## Description

### TECHNICAL FIELD

The present invention relates to a dilator.

### BACKGROUND ART

Dilators are known for expanding a hole formed on the wall of the patient's digestive tract and the like for the purpose of treatment. The front end of a dilator is inserted into a hole formed on a wall, and a tapered portion is then pushed into the hole to expand the hole. Such a dilator is disclosed in, for example, JP 2008-11867 A, WO 91/07202 A1, US 2002/077655 A1, US, 5630813, US 2011/098531 A1, US 2014/046357 A1, and DE 19636318 A1.

Specifically, WO 91/07202 A1 discloses a dilator with a hollow shaft and a grip portion, wherein the shaft includes a tapered portion having an outer diameter of a front end smaller than that of a base end, and wherein a spirally-arranged protruding portion is provided on an outer surface of the shaft, which has gaps between adjacent portions along an axis of the shaft. The spirally-arranged protruding portion is formed of a continuous helical screw thread of increasing pitch and tread height from a fine pitch and low height portion on a tapered distal end portion, to a gross pitch and high height portion on a tubular portion.

Further, US 2002/077655 A1 discloses a dilator with a thread protruding from an outer peripheral surface of a tapered front end tip of a hollow shaft, wherein the thread may have different thread pitches and be formed such that it cuts automatically.

### SUMMARY OF INVENTION

### TECHNICAL PROBLEM

When the dilator as described above is very long, a rotational force and a pushing force from the hand side may not be well transmitted to the front end of the dilator, and thus a hole formed on the wall of the digestive tract cannot be sufficiently expanded.

Starting from WO 91/07202 A1, an object of the present invention is to provide a dilator with a hollow shaft having a spirally-arranged protruding portion which can be easily formed.

### SOLUTION TO PROBLEM

The above object is solved by a dilator in accordance with claim 1. Preferred embodiments thereof are subject-matter of dependent claims.

Further, the shaft may include a first coil having a wire wound around into a hollow shape, with the coil of the spirally-arranged protruding portion forming a second coil.

Moreover, if the shaft includes the first coil including a wire wound around into a hollow shape, and the spirally-arranged protruding portion includes the second coil having a wire wound around on the outer peripheral surface of the shaft, the wires of the first coil and the second wire being wound around in a direction opposite to each other.

### ADVANTAGEOUS EFFECTS OF INVENTION

The present invention can provide a dilator capable of easily increasing the diameter of a hole formed on the wall of the digestive tract and the like.

### BRIEF DESCRIPTION OF DRAWINGS

Fig. 1 shows an overall view of a dilator according to a first embodiment of the present invention.
Fig. 2 shows a front-end side portion of a dilator according to a second embodiment.

### DESCRIPTION OF EMBODIMENTS

Below, the embodiments of the present invention will be described with reference to the figures. It is noted that the dimensions of dilators shown in the figures are provided to merely facilitate understanding of the embodiments, but do not necessarily correspond to the actual dimensions.

### First embodiment

A first embodiment of the present invention will be described with reference to the figure.

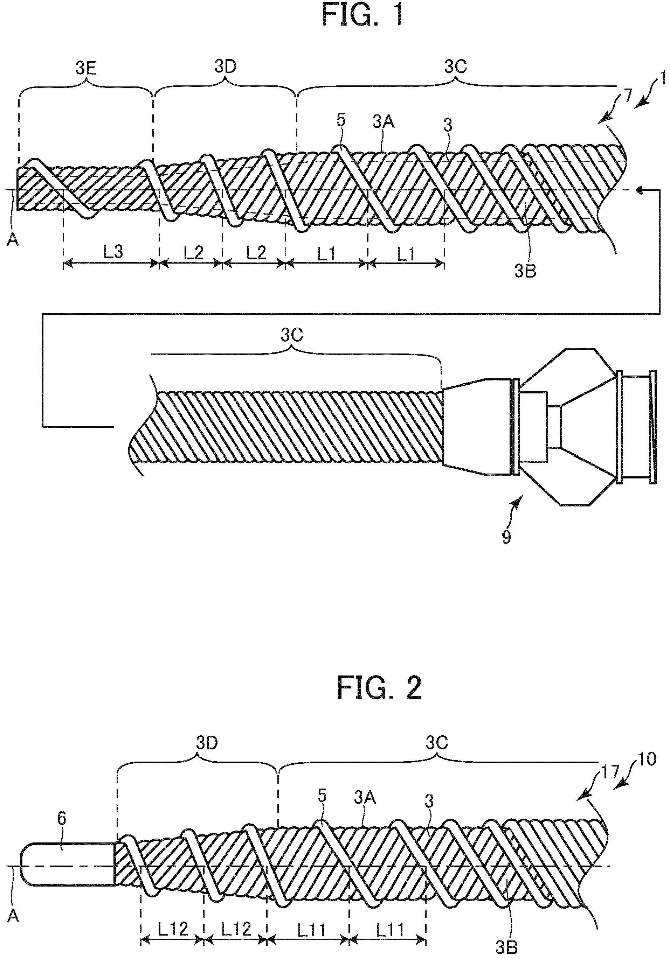

Fig. 1 shows an overall view of a dilator 1 according to the first embodiment of the present invention.

In Fig. 1, the left side in the figure corresponds to the front end side (the distal side) which is to be inserted into the body, and the right side corresponds to the base end side (the hand side, the proximal side) which is to be operated by an operator such as a surgeon.

In Fig. 1, the dilator 1 includes a multilayer body 7 including a first coil 3 having a plurality of metal wires wound around into a hollow shape and a second coil 5 having a single metal wire wound around on an outer peripheral surface 3A of the first coil 3 in a direction (clockwise, facing to the front end) opposite to the first coil 3 (counterclockwise, facing to the front end); and a connector 9 having a hollow shape and connected to a base end of the multilayer body 7.

The wires of the first coil 3 and the second coil 5 are, for example, metal wires of stainless steel, a superelastic alloy such as nickel-titanium, and the like, or of resin wires.

The first coil 3 is configured such that metal wires of, for example, 10 stainless steel wires are wound around. The first coil 3 has a hollow shape in which an inner cavity 3B is formed penetratingly extending from a base end to a front end. The first coil 3 has a base end portion 3C, a tapered portion 3D, and a front end portion 3E. The first coil 3 corresponds to a shaft.

The base end portion 3C is located in the base end side of the dilator 1, and the connector 9 is connected to a base end thereof. Further, the base end portion 3C has a substantially constant outer diameter from the base end thereof through a front end.

The tapered portion 3D is located at the front end side of the base end portion 3C, and extends to the front end side from the front end of the base end portion 3C, and is configured so as to have an outer diameter is smaller toward the front end side.

The front end portion 3E is located at the front end side of the tapered portion 3D, and extends to the front end side from a front end of the tapered portion 3D. The front end portion 3E has a substantially constant outer diameter from a base end thereof through a front end. As described above, the first coil 3 which corresponds to a shaft has a hollow shape having an outer diameter of a front end smaller than that of a base end.

The second coil 5 has, for example, a single metal wire wound around on the outer peripheral surface 3A of the first coil 3 in a direction (clockwise, facing to the front end) opposite to the first coil 3 (counterclockwise, facing to the front end). Here, the metal wire is wound around closely at the base end side, and wound around with gap between adjacent windings at the front end side of the base end portion 3C, the tapered portion 3D, and the front end portion 3E. A portion of the second coil 5 wound around with gap provides a spirally-arranged protruding portion protruding outwardly (outermost portion of the dilator 1, the outermost surface) on the outer peripheral surface 3A of the first coil 3. The above spirally-arranged protruding portion has gaps between adjacent portions (adjacent portions of a metal wire) along an axis A of the first coil 3. The screw action of the above spirally-arranged protruding portion enables the dilator 1 to be moved forward even by a rotational operation of the dilator 1.

Further, pitches of adjacent portions along the axis A of the second coil 5 provided on the tapered portion 3D are configured to be smaller than those of adjacent portions along the axis A of the second coil 5 provided on the base end portion 3C and the front end portion 3E. That is, the configuration is such that L1, L3 > L2 for the adjacent portions along the axis A of the second coil 5 wherein L1 represents a pitch at the front end side of the base end portion 3C, and L2 represents a pitch at the tapered portion 3D, and L3 represents a pitch at the front end portion 3E. It is noted that the pitches at the front end side of the base end portion 3C may be same or different. The pitches at the tapered portion 3D may be same or different.

Further, with regard to the metal wire of the second coil 5, the amount of gap between adjacent portions of the metal wire is gradually decreased toward the base end side thereof at the base end portion 3C. This configuration enables the stiffness of the dilator 1 (the multilayer body 7) along the axis direction to be gradually changed so that the dilator 1 can easily enter into an approach pathway even when the pathway meanders.

In the present embodiment and other embodiments described hereinafter, the length of a dilator is, for example, 2000 mm, preferably 1600 mm to 2500 mm, and the length of the front end portion 3E is, for example, 10 mm, preferably 0 to 100 mm, and the length of the tapered portion 3D is, for example, 30 mm, preferably 5 to 100 mm. The inner diameter of the front end of the first coil 3 is, for example, 0.7 mm, preferably 0.4 to 1.0 mm, and the inner diameter of the base end of the first coil 3 is, for example, 1.5 mm, preferably 1.0 to 3.0 mm. The outer diameter of the front end of the second coil 5 is, for example, 1.84 mm, preferably 0.8 to 3.0 mm, and the outer diameter of the base end of the second coil 5 is, for example, 2.64 mm, preferably 1.4 mm to 5.0 mm. Further, the diameters of the metal wires of the first coil 3 are, for example, 0.21 mm, preferably 0.1 to 0.5 mm, and the diameter of the metal wire of the second coil 5 is, for example, 0.36 mm, preferably 0.1 to 0.5 mm.

Further, the pitches L1 and L3 of the second coil 5 at the base end portion 3C and the front end portion 3E are, for example, 2 mm, the pitch L2 of the second coil 5 at the tapered portion 3D is, for example, 1.5 mm. The ratio (L2/L1 or L3) of them is 0.75. It is noted that the pitches L1 and L3 of the second coil 5 at the base end portion 3C and the front end portion 3E are preferably 0.25 to 5 mm, and the pitch L2 of the second coil 5 at the tapered portion 3D is preferably 0.2 to 4 mm, and the ratio of them ranges between 0.04 and 1.

The connector 9 as a grip portion is a portion through which an operator pushes the dilator into the body, and/or performs a rotational operation. The connector 9 has a front end connected to the base end of the first coil 3 and the base end of the second coil 5. The connector 9 is made of a resin, and has a hollow shape having an inner cavity in communication with the inner cavity 3B of the first coil 3.

In the dilator 1 according to the present embodiment, the spirally-arranged protruding portion (the second coil 5) protruding outwardly is provided on the outer peripheral surface 3A of the first coil 3 which corresponds to a shaft, and has gaps between adjacent portions along the axis direction of the first coil 3. This configuration enables the dilator to be moved forward not only by a conventional pushing operation, but also by a rotational operation of the spirally-arranged protruding portion.

Further, the pitches of adjacent portions along the axis A of the second coil 5 provided on the tapered portion 3D are configured to be smaller than those of adjacent portions along the axis A of the second coil 5 provided on the base end portion 3C and the front end portion 3E. By virtue of this configuration, the frictional resistance with a target matter (for example, the digestive tract such as stomach, and liver) is larger at the tapered portion 3D of the first coil 3 than at the base end portion 3C and the front end portion 3E when the dilator 1 is rotated. This enables a driving force to be larger at the tapered portion 3D than at the base end portion 3C and the front end portion 3E of the dilator 1, leading to easy expansion of a hole.

Further, a shaft comprised of the first coil 3 including a plurality of metal wires wound around into a hollow shape can improve the flexibility of the shaft and the transmissibility of torque via the first coil 3. Further, a spirally-arranged protruding portion comprised of the second coil 5 having a single metal wire wound around on the outer peripheral surface 3A of the first coil 3 can be easily formed, and can ensure the flexibility of the front end of the dilator 1 by virtue of the elasticity of the second coil 5, and can improve the torquability. Further, wires of the first coil 3 and the second coil 5 are wound around in a direction opposite to each other. Therefore, even when the dilator 1 is rotated in a direction to open the first coil 3, a force is applied in a direction to close the second coil 5 to prevent opening of the first coil 3. This allows a force applied to the connector 9 of the dilator 1 to be transmitted to the front end side.

Next, an example of operating modes of the above dilator will be described.

First, a target matter is punctured with an introducer needle to open a hole. Subsequently, a guide wire is inserted into an inner cavity of the introducer needle, and then the introducer needle is withdrawn.

Next, the base end of the guide wire is inserted into an inner cavity of the above dilator, and then the dilator is inserted. Subsequently, the dilator is pushed in while rotating the shaft to expand the hole at a punctured portion. During this, the tapered portion moves forward by virtue of the screw action of the spirally-arranged protruding portion by a rotational operation of the shaft and others, enabling the tapered portion to smoothly expand the hole.

### Second embodiment

Fig. 2 shows a front-end side portion of a dilator 10 according to a second embodiment.

In Fig. 2, the left side in the figure corresponds to the front end side (the distal side) which is to be inserted into the body, and the right side corresponds to the base end side (the hand side, the proximal side) which is to be operated by an operator such as a surgeon.

It is noted that the dilator 10 according to the present embodiment basically has the same structure as the dilator 1 according to the first embodiment. Therefore, the same number is given to the same member, and detailed description will be omitted.

In Fig. 2, the dilator 10 includes a multilayer body 17 including the first coil 3 including a plurality of metal wires wound around into a hollow shape and the second coils 5 having a single metal wire wound around on the outer peripheral surface 3A of the first coil 3 in a direction (clockwise, facing to the front end) opposite to the first coil 3 (counterclockwise, facing to the front end); and the connector 9 having a hollow shape and connected to a base end of the multilayer body 17. However, the dilator 10 differs from the dilator 1 in that the dilator 10 has a forefront portion 6 instead of the front end portion 3E of the first coil 3 of the dilator 1. According to the present embodiment, the first coil 3 having the forefront portion 6 provided at the front end corresponds to a shaft.

The forefront portion 6 is formed by casting a solder material (a silver-tin solder material, a gold-tin solder material, and the like) into the front end of the first coil 3, and has a substantially tubular hollow shape. Further, the forefront portion 6 has a flat surface while the front end of the multilayer body 7 has an uneven surface.

Again in the present embodiment, the pitches of adjacent portions along the axis A of the second coil 5 provided on the tapered portion 3D are configured to be smaller than those of adjacent portions along the axis A of the second coil 5 provided on the base end portion 3C. That is, the configuration is such that L11 > L12 for the adjacent portions along the axis A of the second coil 5 wherein L11 represents a pitch at the front end side of the base end portion 3C, and L12 represents a pitch at the tapered portion 3D.

The dilator 10 having this configuration can produce similar effects as the dilator 1 according to the first embodiment. That is, the frictional resistance with a target matter (for example, the digestive tract such as stomach, and liver) is larger at the tapered portion 3D of the first coil 3 than at the base end portion 3C when the dilator 10 is rotated. This enables a driving force to be larger at the tapered portion 3D than at the base end portion 3C of the dilator 10, leading to easy expansion of a hole. Further, the forefront portion 6 having a flat surface is connected to the front end of the multilayer body 17. This configuration can further improve insertability into a punctured portion by first pressing the dilator against the punctured portion, and then pushing and rotating the dilator thereinto.

Hereinbefore, the embodiments of the present invention are described, but the present invention shall not be limited to these embodiments. Rather, various modifications may be made.

For example, the first coil 3 is described as a follow coil body including 10 wires in the aforementioned embodiments, but the number of wires shall not be limited to 10. The number may be one or more. Further, the second coil 5 is described as a follow coil body including a single wire in the aforementioned embodiments, but the number of wires shall not be limited to 1. The number may be one or more.

Further, the forefront portion 6 according to the second embodiment is described to be formed by casting a solder material into the front end of the multilayer body 17. However, the outer periphery of the second coil 5 and/or the first coil 3 in the vicinity of the front end portion of the multilayer body 17 may be ground to form the forefront portion 6 having a flat surface.

Furthermore, the forefront portion 6 in the second embodiment is fixed to the front end of the multilayer body 17, but the forefront portion may be fixed to the front end of the shaft 21 according to the third and fifth embodiments, or may be fixed to the front end of the shaft 31 according to the fourth and sixth embodiments.

Further, the outer peripheries of the multilayer bodies 7 and 17 according to the first to second embodiments may be coated with a resin(s).

In the embodiments shown in Figs. 1 and 2, shown are dilators each including a shaft having no surface coating. However, the shaft may have various types of coating on the side of the surface thereof (including a portion between the shaft and the spirally-arranged protruding portion). Examples of the coating include, for example, a protective film on the surface of the shaft (representative example: a plating film), an underlying film for improving adhesiveness between the shaft and the spirally-arranged protruding portion, and the like.

Preferably, the spirally-arranged protruding portions according to the embodiments as shown in Figs. 1 and 2 are not configured to serve as a blade. The dilators according to the present embodiments are intended for expanding a hole pre-formed on a target matter (for example, the wall of the digestive tract such as the patient's stomach). Therefore, if the spirally-arranged protruding portion serves as a blade, living body tissues at the inner surface of the hole may be damaged.

For this reason, the spirally-arranged protruding portion preferably does not have a sharp edge at an end portion in a radially outer side of the shaft on a cross-section. That is, the above end portion preferably has an area having a shape including an obtuse edge or a curve (for example, a curve constituting a part of a circle or an ellipse).

### REFERENCE SIGNS LIST

- 1, 10: Dilator
- 3: First coil
- 3A: Outer peripheral surface
- 5: Second coil
- 9: Connector
- 3D: Tapered portion
- 3C: Base end portion
- L1, L2, L3, L11, L12, L13: Pitch

## Claims

1. A dilator (1; 10) comprising:
a hollow shaft (3); and
a grip portion (9) provided on a base end of the shaft (3),
the shaft (3) including:
a tapered portion (3D) having an outer diameter of a front end smaller than that of a base end; and
a base end portion (3C) provided at a base end side of the tapered portion (3D) and extending to the base end side and a front end portion (3E) provided at a front end side of the tapered portion (3D) and extending to the front end side; or
a base end portion (3C) provided at a base end side of the tapered portion (3D) and extending to the base end side, without the front end portion (3E), wherein,
a spirally-arranged protruding portion (5) is provided on an outer peripheral surface (3A) of the shaft (3),
the spirally-arranged protruding portion (5) has gaps between adjacent portions along an axis (A) of the shaft (3), and
in a case where the shaft (3) does not include the front end portion (3E), pitches of adjacent portions along the axis (A) of the spirally-arranged protruding portion (5) provided on the tapered portion (3D) are smaller than those of adjacent portions along the axis (A) of the spirally-arranged protruding portion (5) provided on the base end portion, and
in a case where the shaft (3) includes the front end portion (3E), the pitches of adjacent portions along the axis (A) of the spirally-arranged protruding portion (5) provided on the tapered portion (3D) are smaller than those of adjacent portions along the axis (A) of the spirally-arranged protruding portion (A) provided on the front end portion (3E) or the base end portion (3C), **characterized in that**
the spirally-arranged protruding portion (5) includes a coil having a wire wound around on the outer peripheral surface (3A) of the shaft (3).

2. The dilator (1) according to claim 1, wherein
in a case where the shaft (3) includes the front end portion (3), the pitches of adjacent portions along a direction of the axis (A) of the spirally-arranged protruding portion (5) provided on the tapered portion (3D) are smaller than both those of adjacent portions along the axis direction of the spirally-arranged protruding portion (5) provided at the front end portion (3E) and those of adjacent portions along the direction of the axis (A) of the spirally-arranged protruding portion (5) provided on the base end portion (3C).

3. The dilator (1; 10) according to claim 1 or 2, wherein
the shaft (3) includes a first coil having a wire wound around into a hollow shape, and
the coil (5) of the spirally-arranged protruding portion forms a second coil.

4. The dilator (1; 10) according to claim 3, wherein,
the wires of the first coil (3) and the second coil (5) are wound around in a direction opposite to each other.

## Patentansprüche

1. Dilatator (1; 10) mit:
einem hohlen Schaft (3); und
einem an einem hinteren Ende des Schafts (3) vorgesehenen Griffteil (9),
wobei der Schaft (3) aufweist:
einen verjüngten Abschnitt (3D), der an einem vorderen Ende einen kleineren Durchmesser hat als an dem hinteren Ende; und
einen auf der Seite des hinteren Endes des verjüngten Abschnitts (3D) vorgesehenen hinteren Endabschnitt (3C), der sich zur Seite des hinteren Endes erstreckt, und einen auf der Seite eines vorderen Endes des verjüngten Abschnitts (3D) vorgesehenen vorderen Endabschnitt (3E), der sich zur Seite des vorderen Endes erstreckt; oder
einen auf der Seite des hinteren Endes des verjüngten Abschnitts (3D) vorgesehenen hinteren Endabschnitt (3C), der sich zur Seite des hinteren Endes erstreckt, ohne den vorderen Endabschnitt (3E), wobei
an einer Außenumfangsfläche (3A) des Schafts (3) ein spiralartig angeordneter Vorsprung (5) vorgesehen ist,
der spiralartig angeordnete Vorsprung (5) zwischen benachbarten Abschitten entlang einer Achse (A) des Schafts (3) Lücken aufweist, und
wenn der Schaft (3) den vorderen Endabschnitt (3E) nicht aufweist, die Abstände entlang der Achse (A) zwischen benachbarten Abschnitten des an dem verjüngten Abschnitt (3D) vorgesehenen spiralartig angeordneten Vorsprungs (5) kleiner sind als die Abstände entlang der Achse (A) zwischen benachbarten Abschnitten des an dem hinteren Endabschnitt vorgesehenen spiralartig angeordneten Vorsprungs 5), und
wenn der Schaft (3) den vorderen Endabschnitt (3E) aufweist, die Abstände entlang der Achse (A) zwischen benachbarten Abständen des an dem verjüngten Abschnitt (3D) vorgesehenen spiralartig angeordneten Vorsprungs (5) kleiner sind als die Abstände entlang der Achse (A) zwischen benachbarten Abschnitten des an dem vorderen Endabschnitt (3E) oder an dem hinteren Endabschnitt (3C) vorgesehenen spiralartig angeordneten Abschnitts (5), **dadurch gekennzeichnet, dass**
der spiralartig angeordnete Vorsprung (5) eine Wicklung mit einem um die Außenumfangsfläche (3A) des Schafts (3) gewickelten Draht aufweist.

2. Dilatator (1) nach Anspruch 1, wobei
wenn der Schaft (3) den vorderen Endabschnitt (3) aufweist, die Abstände entlang der Achse (A) zwischen benachbarten Abschnitten des an dem verjüngten Abschnitt (3D) vorgesehenen spiralartig angeordneten Vorsprungs (5) kleiner sind als die Abstände entlang der Achse zwischen benachbarten Abschnitten des an dem vorderen Endabschnitt (3E) vorgesehenen spiralartig angeordneten Abschnitts (5) und die Abstände entlang der Achse (A) zwischen benachbarten Abschnitten des an dem hinteren Endabschnitt (3C) vorgesehenen spiralartig angeordneten Abschnitts (5).

3. Dilatator (1; 10) nach Anspruch 1 oder 2, wobei
der Schaft (3) eine erste Wicklung mit einem in eine hohle Form gewickelten Draht aufweist, und
die Wicklung (5) des spiralartig angeordneten Vorsprungs eine zweite Wicklung bildet.

4. Dilatator (1; 10) nach Anspruch 3, wobei
die Drähte der ersten Wicklung (3) und der zweiten Wicklung (5) in entgegengesetzte Richtungen gewickelt sind.

## Revendications

1. Dilatateur (1 ; 10) comprenant :
un arbre creux (3) ; et
une partie de préhension (9) prévue sur une extrémité de base de l'arbre (3),
l'arbre (3) comprenant :
une partie effilée (3D) ayant un diamètre extérieur d'une extrémité avant plus petit que celui d'une extrémité de base ; et
une partie d'extrémité de base (3C) prévue sur un côté d'extrémité de base de la partie effilée (3D) et s'étendant vers le côté d'extrémité de base et une partie d'extrémité avant (3E) prévue sur un côté d'extrémité avant de la partie effilée (3D) et s'étendant vers le côté d'extrémité avant ; ou
une partie d'extrémité de base (3C) prévue sur un côté d'extrémité de base de la partie effilée (3D) et s'étendant vers le côté d'extrémité de base, sans la partie d'extrémité avant (3E), dans lequel,
une partie saillante disposée en spirale (5) est prévue sur une surface périphérique extérieure (3A) de l'arbre (3),
la partie saillante disposée en spirale (5) présente des espaces entre des parties adjacentes le long d'un axe (A) de l'arbre (3), et
dans un cas où l'arbre (3) ne comprend pas la partie d'extrémité avant (3E), des pas de parties adjacentes le long de l'axe (A) de la partie saillante disposée en spirale (5) prévue sur la partie effilée (3D) sont plus petits que ceux des parties adjacentes le long de l'axe (A) de la partie saillante disposée en spirale (5) prévue sur la partie d'extrémité de base, et
dans le cas où l'arbre (3) comprend la partie d'extrémité avant (3E), les pas des parties adjacentes le long de l'axe (A) de la partie saillante disposée en spirale (5) prévue sur la partie effilée (3D) sont plus petits que ceux des parties adjacentes le long de l'axe (A) de la partie saillante disposée en spirale (A) prévue sur la partie d'extrémité avant (3E) ou la partie d'extrémité de base (3C), **caractérisé en ce que**
la partie saillante disposée en spirale (5) comprend une bobine ayant un fil enroulé autour de la surface périphérique extérieure (3A) de l'arbre (3).

2. Dilatateur (1) selon la revendication 1, dans lequel
dans un cas où l'arbre (3) comprend la partie d'extrémité avant (3), les pas des parties adjacentes le long d'une direction de l'axe (A) de la partie saillante disposée en spirale (5) prévue sur la partie effilée (3D) sont plus petits que ceux des parties adjacentes le long de la direction d'axe de la partie saillante disposée en spirale (5) prévue sur la partie d'extrémité avant (3E) et ceux des parties adjacentes le long de la direction de l'axe (A) de la partie saillante disposée en spirale (5) prévue sur la partie d'extrémité de base (3C).

3. Dilatateur (1 ; 10) selon la revendication 1 ou 2, dans lequel
l'arbre (3) comprend une première bobine ayant un fil enroulé autour en une forme creuse, et
la bobine (5) de la partie saillante disposée en spirale forme une seconde bobine.

4. Dilatateur (1 ; 10) selon la revendication 3, dans lequel,
les fils de la première bobine (3) et de la seconde bobine (5) sont enroulés dans une direction opposée l'un à l'autre.
